# EUROPEAN PATENT SPECIFICATION

(11) **EP 0 711 765 B1**
(45) Date of publication and mention of the grant of the patent: **27.08.2003**
(21) Application number: 95308102.3
(22) Date of filing: 13.11.1995
(51) Int. Cl.: C07D 307/92, C07D 493/04, C07D 491/04, C07D 498/04, A61K 31/365, A61K 31/415, A61K 31/42

(54) **Immunosuppressive cyclolignan derivatives**
Immmunosuppressive Cyclolignanderivate
Dérivés de cyclolignane possédant und activité immunosuppressive

(30) Priority: 14.11.1994 GB 9422947
(43) Date of publication of application: 15.05.1996
(73) Proprietor: UNIVERSIDAD DE SALAMANCA, 37007 Salamanca (ES)
(72) Inventor: Gordaliza, Marina, E-37007 Salamanca (ES); Castro, Maria Angeles, E-37007 Salamanca (ES); San Feliciano, Arturo, E-37007 Salamanca (ES); del Corral, Jose Maria Miguel, E-37007 Salamanca (ES); Lopez, Maria Luisa, E-37007 Salamanca (ES); Faircloth, Glynn T. Pharma Mar S.A., E-28670 Tres Cantos, Madrid (ES)
(74) Representative: Ruffles, Graham Keith

(56) References cited:
- WO-A-86/04062
- DE-A- 3 612 278
- US-A- 4 567 253
- ACTA CHEMICAL SCANDINAVICA, vol. 47, no. 12, December 1993 COPENHAGEN DK, pages 1190-1200, H.F. HANSEN ET AL. 'New Compounds Related to Podophyllotoxin and Congeners: Synthesis, Structure Elucidation and Biological Testing'
- JOURNAL OF THE CHEMICAL SOCIETY, PERKIN TRANSACTIONS 1, no. 21, 7 November 1993 LETCHWORTH GB, pages 2541-2548, D.W. JONES ET AL. 'Synthesis of (+-)-4-Deoxypodophyllotoxin, (+-)-Podophyllotoxin and (+-)-Epipodphyllotoxin'
- JOURNAL OF PHARMACEUTICAL SCIENCES, vol. 75, no. 11, November 1986 WASHINGTON US, pages 1076-1080, O. BUCHARDT ET AL. 'Thermal Chemistry of Podophyllotoxin in Ethanol and a Comparison of the Cytostatic Activity of the Thermolysis Products'
- JOURNAL OF ORGANIC CHEMISTRY, vol. 54, no. 18, 1 September 1989 EASTON US, pages 4280-4290, S.P. FORSEY ET AL. 'Comprehensive Synthetic Route to Eight Diastereomeric Podophyllum Lignans'
- CHEMICAL ABSTRACTS, vol. 120, no. 19, 9 May 1994 Columbus, Ohio, US; abstract no. 235424b, M. GORDALIZA ET AL. 'Antineoplastic and antiviral activities of podophyllotoxin related lignans' page 26; column 1; & ARCH. PHARM., vol. 327, no. 3, 1994 pages 175-179,
- CHEMICAL AND PHARMACEUTICAL BULLETIN, vol. 40, no. 10, October 1992 TOKYO JP, pages 2720-2727, T. TERADA ET AL. 'DNA Topomerase II Inhibitory Activity and the Structural Relationship of Podophyllotoxin Derivatives as Antitumor Agents'
- JOURNAL OF MEDICINAL CHEMISTRY, vol. 32, no. 3, March 1986 WASHINGTON US, pages 604-608, L.S. THURSTON ET AL. 'Antitumor Agents. 100. Inhibition of Human DNA Topoisomerase II by Cytotoxic Ether and Ester Derivatives of Podophyllotoxin and alpha-Peltatin'
- SCHREIER E.: '165. Zur Struktur des Sikkimotoxins II. Partialsynthese der 6,7-Dimethoxy-Analogen von Podophyllotoxin, Epi-, Neo- und Desoxy-podophyllotoxin' HELVETICA CHIMICA ACTA vol. 47, no. 6, 1964, BASEL, CH, pages 1529 - 1555
- SCHREIER E.: '9. Zur Struktur des Sikkimotoxins I. Synthese von stereoisomeren 6,7-Dimethoxy-Analogen des Podophyllotoxins' HELVETICA CHIMICA ACTA vol. 46, no. 1, 1963, BASEL, CH, pages 75 - 115

## Description

This invention is concerned with cyclolignan derivatives, the preparation of such derivatives, and pharmaceutical compositions containing them.

Podophyllotoxin is a known cyclolignan derivative with antimitotic and related activities, see for example the discussion in US 4,567,253. Many derivatives have been discovered or synthesised, see additionally for example Acta Chem. Scan. 1993 47:1190 and Arch. Pharm. (Weinheim) 327, 175 (1994). WO 86/04062 reports an effect against reactions of immunity and rejection in respect of podophyllotoxin.

Helvetica Chimica Acta, **46** (1), 75(1963) and **47** (6), 164(1964) disclose the compounds hereinafter defined as AP-39 and AP-40 and a method for synthesising them.

Chem.Pharm.Bull. **40**(10), 2720 (1992) discloses the compound hereinafter defined as AP-37 and other podophyllotoxin derivatives which are stated to have antitumour activity. However, according to this document, the compounds used in the present invention show neither inhibition of tubulin polymerisation nor inhibition of DNA topoisomerase II.

DE-A-3612278 discloses epipodophyllotoxin and related compounds of use as antineoplastic agents. The definition in this publication for the group R⁵ does not coincide with the compounds of the present invention.

J.Pharm.Sci., **75** (11) 1076 (1986) relates to podophyllotoxin and this thermolysis products. J.Org.Chem., **54** (18), 4280 (1989) relates to intermediates for podophyllotoxin synthesis. Chem.Abs. **120** 235424b (1996) relates to various podophyllotoxin derivatives with antineoplastic and antiviral activities. J.Chem.Soc.Perkin Trans. 1, **21,** 2541 (1993) is concerned with the synthesis of various podophyllotoxin derivatives. It has now been found, in accordance with the present invention, that the above known cyclolignan derivatives, as hereinafter defined, possess immunomodulatory activity, as evidenced by both in vitro (MLR) and in vivo (GVHR and SG) tests.

Accordingly, the invention provides the use of cyclolignan derivatives of the formula (I): 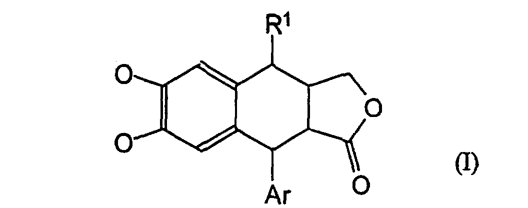 in which:
R¹ is hydrogen or hydroxy; and
Ar is 3,4,5-trialkoxyphenyl, wherein each alkoxy moiety may be the same or different and each has from 1 to 6 carbon atoms;
in the manufacture of an immunomodulatory pharmaceutical composition.

### PREFERRED EMBODIMENTS

The alkoxy groups have from 1 to 6 carbon atoms, more preferably 1 to 4 carbon atoms. Examples include methoxy, ethoxy, propoxy, isopropoxy, butoxy, t-butoxy and other alkoxy groups.

The present compounds can exist as optical isomers, and the invention embraces the individual isomers and mixtures thereof, including the diastereomeric or racemic mix. The stereochemistry of the substituents may be selected as desired, and for example the group R¹ can be α or β.

Individual preferred compounds usable in this invention include
AP-37, which is 3,4-O-demethylenedeoxypicropodophyllin;
AP-39, which is 3,4-O-demethyleneepipodophyllotoxin; and
AP-40, which is 3,4-O-demethylenepodophyllotoxin.
Compounds of formula (I), as noted above, have immunomodulatory activity. Accordingly, immunosuppressive pharmaceutical compositions comprising compounds of formula (I) as defined above in association with a pharmaceutical carrier, can be readily envisaged by those skilled in the art. Such pharmaceutical compositions may, for example, be adapted for oral, parenteral, or rectal administration by the incorporation or appropriate pharmaceutical carriers and options adjuvants.
   The immunosuppressive activity of a compound in accordance with the invention is summarised below.

### Immunosuppressive effects of several representative cyclolignans

| Compound | dose^{a} | GVHR index^{b} | SG index^{c} | Cytotox.^{d} |
|---|---|---|---|---|
| AP-37 | 0.1 | 58 | -- | >20 |
| AP-39 | 1.5 | -- | 200 | >10 |
| AP-40 | 1.5 | -- | 229 | 0.5 |
| Cyclophosphamide | 200 | 79 | -- | -- |
| Cyclosporin A | 25 | -- | 178^{e} | -- |

| | | | | |
|---|---|---|---|---|
| a. mg/kg/day (7 days) | | | | |
| b. % reduction of spleen weight with respect to non treated animals. | | | | |
| c. % of implant duration with respect to non treated animals. | | | | |
| d. IC₅₀ (µg/ml) for monkey kidney fibroblasts. | | | | |
| e. 20% of the animals died in this experiment before the end of the program (30 days of observation). | | | | |

The compounds in accordance with the invention may be prepared from podophyllotoxin and analogues formula:

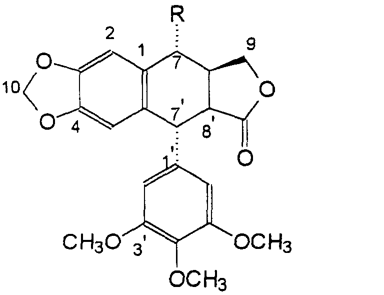

For example, see A.W. Fortschrite Chem. Org. Naturstoff 15. 83 (1953) which describes podophyllotoxin where R is hydroxy and 8'-H is β; deoxypodophyllotoxin where R is hydrogen and 8'-H is β; and deoxypicropodophyllin where R is hydrogen and 8'-H is α. See also J.Med.Chem. 29 1547-1550 (1986) and Chem. Pharm. Bull. 40 2720-2727 (1992).

For example, compounds of this invention can be made in accordance with the following reaction scheme, where partial structures are shown as appropriate: 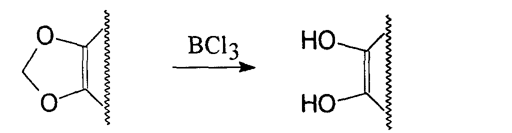 Other equivalent reaction conditions can be employed.

### EXAMPLE OF THE INVENTION

In order that the invention may be further understood, the following example is given by way of illustration only.

### Example

### Preparation of AP-37:

To a solution of boron trichloride in CH₂Cl₂ (1M, 2 ml) precooled at -70° to -65° was added dropwise deoxypicropodophyllin (100 mg) in dry CH₂Cl₂ (6 ml). After stirring at the same temperature for an additional 1 hour, the mixture was poured into ice-water and extracted with ethyl acetate. The residue obtained after evaporating the organic solvent was dissolved in a mixture of water:acetone:calcium carbonate (3ml:3ml:1g) and refluxed for 1 hour. The suspension was acidified with 2N HCl and extracted with ethyl acetate. Evaporation of the solvent afforded 80 mg of AP-37.
[M+]: 386
mp: 226-228°C (MeOH)
[α]_{D}(CHCl₃): +46.8°
UV λₘₐₓ(EtOH)(ε): 222(23300), 288(7200)
IR (KBr): 3400, 1740, 1600, 1510, 1300, 1110 cm⁻¹.

## Claims

1. The use of a compound of the formula (I): 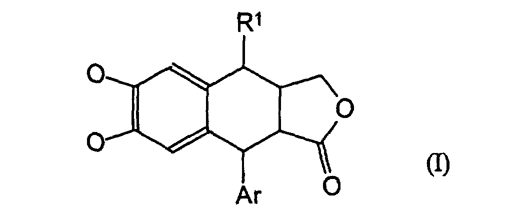 in which:
R¹ is hydrogen or hydroxy; and
Ar is 3,4,5-trialkoxyphenyl, wherein each alkoxy moiety may be the same or different and each has from 1 to 6 carbon atoms,
in the manufacture of an immunomodulatory pharmaceutical composition.

2. The use according to claim 1, wherein the compound is 3,4-O-demethylenedeoxypicropodophyllin.

3. The use according to claim 1, wherein compound is 3,4-O-demethyleneepipodophyllotoxin.

4. The use according to claim 1, wherein the compound is 3,4-O-demethylenepodophyllotoxin.

## Patentansprüche

1. Verwendung einer Verbindung mit der Formel (I) 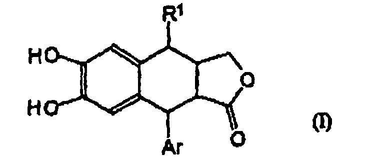 in welcher sind:
R¹ Wasserstoff oder Hydroxy;
Ar 3,4,5-Trialkoxyphenyl steht, wobei eine jede Alkoxyhälfte gleich oder verschieden sein kann und zwischen 1 bis 6 Kohlenstoffatomen aufweist,
für die Herstellung einer immunomodulatorischen pharmazeutischen Zusammensetzung.

2. Verwendung gemäß Anspruch 1, bei welcher die Verbindung aus 3,4-O-Demethylendeoxypicropodophyllin besteht.

3. Verwendung gemäß Anspruch 1, bei welcher die Verbindung aus 3,4-O-Demethylenepipodophyllotoxin besteht.

4. Verwendung gemäß Anspruch 1, bei welcher die Verbindung aus 3,4-O-Demethylenepodophyllotoxin besteht.

## Revendications

1. Utilisation d'une composition ayant la formule (I) 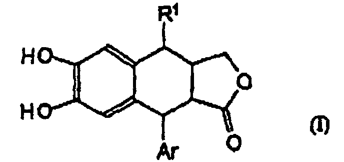 dans laquelle:
R¹ est constitué d'hydrogène ou d'hydroxyle;
Ar est constitué de 3,4,5-trialkoxyphényle, où chaque fraction d'alkoxy peut être identique ou différente et comriporte entre 1 à 6 atomes de carbone,
pour la fabrication d'une composition pharmaceutique immunomodulatoire.

2. Utilisation suivant la revendication 1, pour laquelle la composition est constituée de 3,4-O-déméthylènedésoxypicropodophylline.

3. Utilisation suivant la revendication 1, pour laquelle la composition est constituée de 3,4-O-déméthylèneépipodophyllotoxine.

4. Utilisation suivant la revendication 1, pour laquelle la composition est constituée de 3,4-O-déméthylèneépodophyllotoxine.
